# EUROPEAN PATENT SPECIFICATION

(11) **EP 0 428 613 B1**
(45) Date of publication and mention of the grant of the patent: **20.12.1995**
(21) Application number: 89909794.3
(22) Date of filing: 14.08.1989
(51) Int. Cl.: A61F 6/14, A61F 6/18

(54) **INTRAUTERINE CONTRACEPTIVE DEVICE**
EMPFÄNGNISVERHÜTENDE INTRAUTERINVORRICHTUNG
DISPOSITIF DE CONTRACEPTION INTRA-UTERIN

(30) Priority: 12.08.1988 GB 8819305
(43) Date of publication of application: 29.05.1991
(73) Proprietor: Gardosi, Jason Otto, Dr., Nottingham NG9 3EP (GB)
(72) Inventor: Gardosi, Jason Otto, Dr., Nottingham NG9 3EP (GB)
(74) Representative: Garratt, Peter Douglas
(86) International application number: GB8900935
(87) International publication number: WO9001310

(56) References cited:
- EP-A- 0 117 818
- FR-A- 2 103 284
- US-A- 3 253 590
- US-A- 3 405 711

## Description

This invention relates to gynaecology and more particularly to contraception.

Intrauterine contraceptive devices (IUCD's) are a reliable and popular form of contraception. Most modern designs consist of a polyethylene frame with a coiled copper wire which has a silver core to avoid fragmentation of the copper inside the uterus. Typically, the design is a variation of the shape of a T; the arms stabilise it in the cavity of the uterus and attached to the base of the stem are one or a pair of threads which stay protruding through the cervix of the uterus and are pulled on when the device is to be removed.

The exact method of action of IUCD's is uncertain but their presence is thought to interfere with sperm transport and fertilisation, and implantation of a fertilised ovum.

A number of problems have been identified with existing IUCD designs, these including perforation of the uterus at insertion, contraceptive failure, expulsion, dyspareunia, 'lost' IUCD's and the risk of infection and resultant sterility.

Perforation of the uterus at insertion is an occasional complication and thorough training is required before an operator is confident in the safe placement of IUCD's. Some designs require a complicated 'push-pull-push technique' or similar for proper positioning at insertion. Clearly, the easier the required technique, the less likely the occurence of such a complication.

Contraceptive failure is often thought to be due to incorrect positioning or partial expulsion of the IUCD. Therefore, the device should be designed to be 'fundus seeking' i.e. stay as high up in the fundus of the uterus as possible, as it is here that it has the most contraceptive effect.

Expulsion is a not uncommon problem and it is thought that uterine contractions are a major cause. Many designs have arms with spurs which are aimed to lessen the chance of expulsion.

The threads which are needed for the removal of existing IUCD designs can cause male dyspareunia, which occasionally is a reason for a request to remove the device. A further disadvantage of the thread may be that it 'gives away' to the partner that an IUCD is in situ; this may be a disadvantage, particularly in these cultures where women are struggling for their right to determine whether and which kind of contraception they use.

Sometimes, the thread is 'lost' or taken up into the uterine cavity and is not accessible when removal is intended. In such cases, the device may be retrieved with special instruments, but the device is not designed to be removed without its thread. Therefore, the procedure may be unsuccessful and the patient will need to undergo a 'Dilation and Currettage' under general anaesthesia for the IUCD to be retrieved.

IUDC use has been demonstrated to be associated with pelvic infection in several large studies and it has been suggested that multiplicity of sexual partners and increased risk of sexually transmitted disease is a major risk factor. Studies have shown that ascending colonisation of bacteria into the uterine cavity can occur alongside the thread, whether this is made of mono-or multifilament material. It has been suggested that the thread might interfere with the normal anti-bacterial protective mechanisms of the uterus. Threadless designs existed in the past but they had to be removed after dilatation of the cervix under a general anaesthetic.

Pelvic infection may lead to inflammation of the Fallopian tubes which may result in infertility. The risk of these two complications has been the major reason why IUCD use has drastically dropped, particularly in the U.S.A.

US 3 253 590 which has been used for the delineation of Claim 1 describes a wide variety of shapes which, for the purpose of contraceptive effectiveness, are intended to maximise physical contact between the device and the uterus. This known device belongs to the "foreign-body-reaction" type of IUCDs.

It is an object of this invention to provide an improved intrauterine contraceptive device in which some or all of the above problems are overcome or alleviated.

Accordingly, the present invention consists in an intrauterine contraceptive device consisting only of a resilient closed loop compressible for introduction into the uterus and able to expand towards the uterine wall so as to remain in place within the uterus and to adopt resilient contact with its wall, the device having no thread, characterised in that said loop is carrying a body of contraceptive material.

One feature of this invention is that the device is compressed for introduction into the uterus but then springs open, adoping a closed loop shape, and adapting itself to the shape of the uterus. An associated advantage is that one size of IUCD should fit most uterine cavities as the slack is taken up by this elasticity. This is in contrast to previous, more rigid T-shaped designs. The general closed loop design of the device allows it to be removed by instrumentation through the uterine cervix with a thin forceps by grabbing any part of the loop.

Advantageously, the loop allows itself to be compressed during a uterine contraction, thus decreasing the likelihood of the device being pushed downwards and therefore dislodged or extruded. The energy of uterine compression is thus translated into a resilient compression of the loop and not into dislodgement of the device.

Another feature of this invention is the suitability of the IUCD for use with thin forceps which has jaws that, due to the internal elasticity of its design, open up during protrusion through the uterine cervix, and are open when contact is made with a segment of the closed loop IUCD; a thin canula when advanced over these jaws causes them to close, thus trapping the IUCD. Thus, this manoeuvre involves a simple 'push-push technique'. The operator knows that the device is 'caught' when the forceps are no longer retractible through their canula; the IUCD is then removed by pulling on both the forceps and the canula together.

This invention will now be described by way of example with reference to the accompanying drawings in which:-
Figure 1 shows a front plan view of an IUCD according to this invention inside a model drawing of the uterine cavity;
Figure 2 is a front view of the IUCD of Figure 1 'loaded' into an introducer for insertion into the uterine cavity; and
Figure 3 a,b and c show a front view of forceps for removing the IUCD of Figure 1.

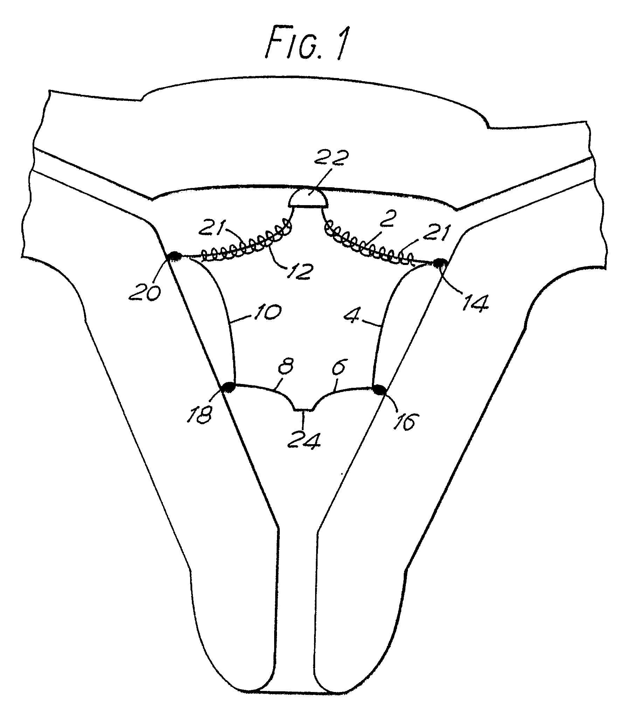

Figure 1 shows an IUCD generally of hexagonal shape which adjusts itself to the shape of the uterine cavity. Limbs 2, 4, 6, 8, 10 and 12 are concave to the uterus wall and nodes 14, 16, 18 and 20 are pressed into resilient contact with the uterus. In this way the nodes serve to keep the device high up in the uterus, near its fundus, and to stabilise the intrauterine position.

The basic structure is made of polyethylene and the limbs 2 and 12 are covered with coiled copper wire. If desired, limbs 4 and 10 may also be covered, thus allowing a large amount of copper to be carried for prolonged contraceptive effectiveness. Other metals or chemicals thought advantageous for contraceptive or therapeutic effect (for example progesterons) can be carried similarly. Button 22 serves to hold together the free ends of limbs 2 and 12. The stirrup 24 formed between limbs 6 and 8 is useful during both insertion and removal of the device, as will be described.

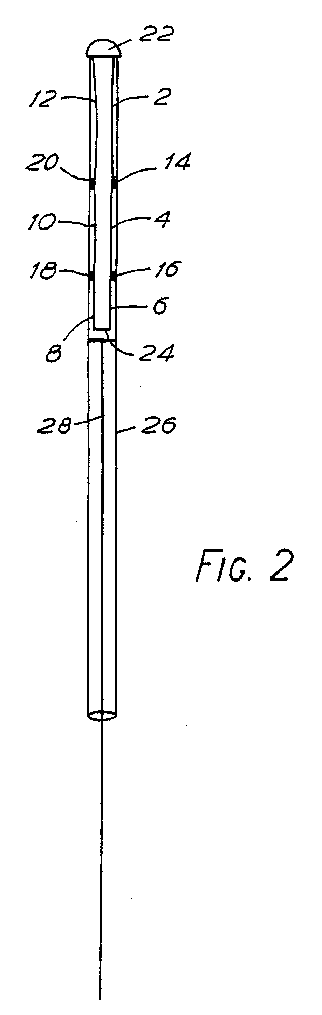

Figure 2 shows the IUCD 'loaded' into an introducer 26 through which it is placed into the uterine cavity, with the help of the trocar 28. During this movement, the trocar 28 engages the stirrup 24 of the IUCD. The button 22 is designed to stop the device from being pushed too far into the introducer during loading, and is a relatively large blunt surface which decreases the chance of uterine perforation during insertion. The absence of a rigid stem in the middle of the IUCD further diminishes the chance of excess pressure being exerted leading to perforation at the time of insertion.

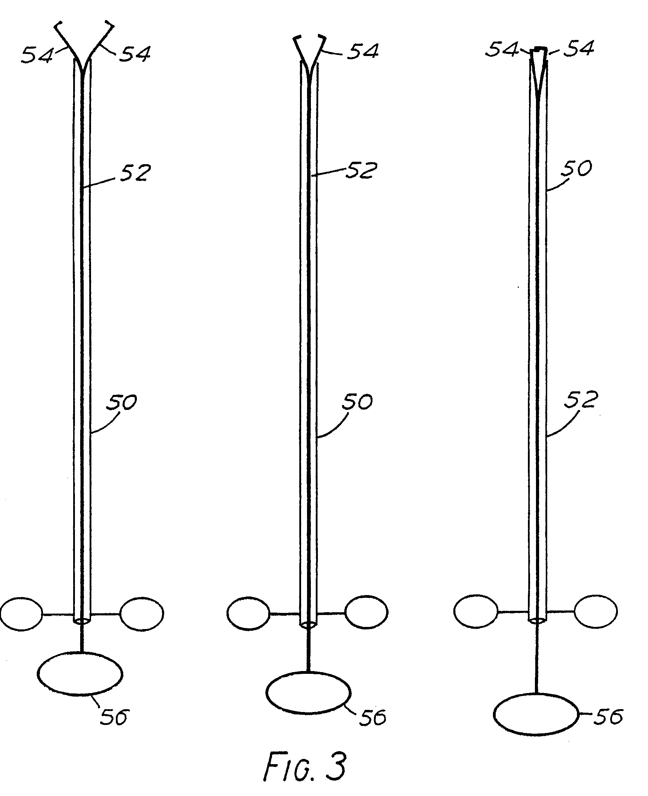

Figure 3 shows schematically how according to one aspect of the invention, forceps grasp and retrieve the IUCD by a 'push-push-pull' technique.

The forceps comprise a canula 50 having a stylet 52 bifurcated to form jaws 54. The opposite end of the stylet 52 is formed with a ring 56.

In the position shown in Figure 3a, the jaws 54 are fully protruded and, made of firm plastic materials, are shaped in such a way that an inherent elasticity causes them to spring apart.

When the stirrup 24 of the IUCD is thought to be between the jaws 54 of the forceps, the canula 50 is advanced, causing the jaws to be brought together for grasping (Fig 3b and c). If pulling on the ring 56 meets resistance, the IUCD is already between the arms and caught. Finally, the forceps and IUCD are removed together.

It should be noted that the IUCD is designed to be stable within the uterine cavity because of its shape similar to the uterine cavity, its elastic properties and the nodes in contact with the uterus wall. However, even if it should rotate within the uterine cavity of the device so that the stirrup 24 is no longer presented to the cervix, the loop design will still enable it to be removed in essentially the same fashion as described above with the forceps grasping whether portion of the loop that faces the cervix.

## Claims

1. An intrauterine contraceptive device consisting only of a resilient closed loop compressible for introduction into the uterus and able to expand towards the uterine wall so as to remain in place within the uterus and to adopt resilient contact with its wall, the device having no thread, characterised in that said loop is carrying a body of contraceptive material.

2. A device according to Claim 1, wherein the body of contraceptive material comprises a coil extending around a portion of the loop.

3. A device according to Claim 1 or Claim 2, wherein the contraceptive material is selected from the group consisting of copper, metals other than copper having contraceptive effectiveness and the progestogens.

4. A device according to any one of the preceding claims, wherein the loop comprises at least three distinct limbs each concave to the uterine wall and interconnected by nodes urged, when the device is in position, into contact with the uterus through resilience in said limbs.

## Patentansprüche

1. Intrauterine empfängnisverhütende Vorrichtung bzw. Intrauterinpessar, die bzw. das nur aus einer elastischen geschlossenen Schleife besteht, die zur Einführung in die Gebärmutter zusammendrückbar ist und dazu in der Lage ist, sich auf die Gebärmutterwand hin zu erstrecken, um so innerhalb der Gebärmutter in Lage zu bleiben und um einen elastischen Kontakt mit ihrer Wand anzunehmen, wobei die Vorrichtung keinen Faden aufweist und **dadurch gekennzeichnet** ist, daß die Schleife einen Körper aus empfängnisverhütenden Material trägt.

2. Vorrichtung gemäß Anspruch 1, bei welcher der Körper aus empfängnisverhütenden Material eine Spule aufweist, die sich um einen Abschnitt der Schleife herum erstreckt.

3. Vorrichtung nach Anspruch 1 oder Anspruch 2, bei welcher das empfängnisverhütende Material aus der Gruppe ausgewählt wird, die aus Kupfer, Metallen, die nicht Kupfer sind, besteht, die eine empfängnisverhütende Wirkungsamkeit und die Progesterone aufweisen.

4. Vorrichtung nach irgendeinem der vorhergehenden Ansprüche, bei welcher die Schleife wenigstens drei verschiedene Glieder aufweist, wobei jedes hohlrund bzw. konkav zur Gebärmutterwand hin ist und durch Knoten verbunden ist, die , wenn die Vorrichtung in Lage ist, durch die Elastizität in den Gliedern in Kontakt mit der Gebärmutter gedrängt werden.

## Revendications

1. Dispositif de contraception intra-utérin consistant seulement en une boucle élastique fermée compressible pour l'introduction dans l'utérus et pouvant se dilater vers la paroi utérine de façon à rester en place dans l'utérus et à être en contact élastique avec sa paroi, le dispositif ne comportant pas de fil, caractérisé en ce que ladite boucle porte un corps de matière contraceptive.

2. Dispositif suivant la revendication 1, dans lequel le corps de matière contraceptive comprend un enroulement s'étendant autour d'une partie de la boucle.

3. Dispositif suivant la revendication 1 ou la revendication 2, dans lequel la matière contraceptive est choisie dans le groupe comprenant le cuivre, les métaux autres que le cuivre ayant un effet contraceptif et les progestogènes.

4. Dispositif suivant une quelconque des revendications précédentes, dans lequel la boucle comprend au moins trois côtés distincts, concaves chacun par rapport à la paroi utérine et interconnectés par des noeuds qui sont pressés, lorsque le dispositif est en position, en contact avec l'utérus par l'élasticité des dits côtés.
